# EUROPEAN PATENT APPLICATION

(11) **EP 2 386 212 A2**
(43) Date of publication of application: **16.11.2011**
(21) Application number: 11163416.8
(22) Date of filing: 21.04.2011
(51) Int. Cl.: A23L 1/226, A23L 1/22, C07C 233/09

(54) **Flavor-enhancing amide compounds**

(30) Priority: 12.05.2010 US 778622
(71) Applicant: INTERNATIONAL FLAVORS & FRAGRANCES INC., New York New York 10019 (US)
(72) Inventor: Bombuwala, Karunananda, New York, 10954 (US); Janczuk, Adam Jan, New Jersey, 08859 (US); John, Thumplasseril V., New Jersey, 07751 (US); Kraut, Kenneth, New Jersey, 07735 (US); Liu, Zhihua, New Jersey, 08816 (US); Mannava, Neelima, Manalapan, NJ 07726 (US); Wu, Hou, East Brunswick, NJ 08816 (US); Yang, Ying, Holmdel, NJ 07733 (US)
(74) Representative: Lawrence, John

(57) **Abstract**

The present invention has discovered that amide compounds represented by Formula I set forth below have unexpected and advantageous flavor enhancement and modification properties: 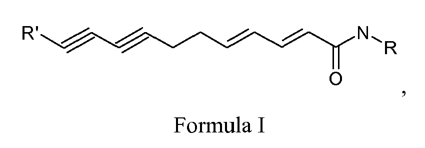 wherein R and R' is independently selected from the group consisting of H and C₁-C₁₀ linear, branched, or cyclic alkyl, alkenyl, alkynyl or aromactic groups. Thus, the amide compounds of the present invention can be used to enhance or modify the flavor of orally consumable compositions, such as foodstuff, chewing gums, dental and oral hygiene products, and medicinal products.

## Description

### Field of the Invention

The present invention relates to the use of amide compounds to enhance or modify the flavor of orally consumable compositions, such as foodstuff, chewing gums, dental and oral hygiene products, and medicinal products.

### Background of the Invention

The term umami, from the Japanese word to describe savory or meaty, is a term used to describe the unique overall fullness and savory taste of food. Materials that exhibit this taste quality generally potentate the intensity of glutamate solutions, which is an important characteristic of the umami taste. Umami is increasingly becoming recognized as the fifth sense of taste, the others being sour, sweet, salt, and bitter. Compounds traditionally described as possessing the umami character are monosodium glutamate (MSG), protein hydrolysates, some amino acids, certain nucleotides, and phosphates.

MSG is the most widely used material as a "taste enhancer" where it synergizes the perception of "savory" ingredients. However, a large amount of MSG may cause adverse effect as well as allergic reactions in human.

Other chemical compounds such as certain nucleotides also exhibit the umami effect, which include adenosine 5'-(trihydrogen diphosphate), 5'-cytidylic acid (5'-CMP), 5'-uridylic acid (5'-UMP), 5'-adenylic acid (5'-AMP), 5'-guanylic acid (5'-GMP), 5'-inosinic acid (5'-IMP), and the di-sodium salts of 5'-guanylic acid and 5'-inosinic acid. Recent literature cites an extensive range of other organic compounds as taste active components of mixtures shown to provide the umami effect. These compounds include but are not necessarily limited to: organic acids such as succinic acid, lactic acid, saturated straight chain aliphatic acids of six, eight, fourteen, fifteen, sixteen, and seventeen carbon chain lengths, Z4,Z7,Z10,Z13,Z16,Z19-docosahexaenoic acid, Z5,Z8,Z11,Z14,Z17-cicosapentaenoic acid, Z9,Z12,Z16,Z19-octadecadienoic acid, Z9-octadecenoic acid, glutaric acid, adipic acid, suberic acid, and malonic acid. Amino acids having umami effect reported in the literature include glutamic acid, aspartic acid, threonine, alanine, valine, histidine, proline, tyrosine, cystine, methionine, pyroglutamic acid, leucine, lycine, and glycine. Dipeptides possessing umami properties include Val-Glu and Glu-Asp. Other miscellaneous compounds having umami properties include alpha-amino adipic acid, malic acid, alpha-aminobutyric acid, alpha-aminoisobutyric acid, E2,E4-hexadienal, E2,E4-heptadienal, E2,E4-octadienal, E2,E4-decadienal, Z4-heptenal, E2,Z6-nonadienal, methional, E3,E5-octadien-2-one, 1,6-hexanediamine, tetramethylpyrazine, trimethylpyrazine, cis-6-dodecen-4-olide, glutamate glycoconjugates, fish sauce blended with anchovy paste (U.S. Patent 7,510,738) and a number of naturally occurring amino acids.

Additionally, a large number of compounds are known to provide a cooling sensation in the mouth, in the nasal cavity, and/or on the skin. The best known and most widely used of these is menthol, which, in addition to olfaction, causes a cooling response on cold receptors in the oral cavity, the nasal cavity, and on the skin. Unfortunately, menthol also exhibits some undesirable properties, such as a strong mint smell, a bitter taste, and relatively high volatility. There still remains a need for novel coolant compositions that provide strong and substantive refreshing and cooling attributes in the absence of negative aroma, negative taste, and negative cooling attributes.

Thus, despite the previous disclosure in the art, there is an ongoing need for novel flavor compounds, particularly those that enhance or modify the umami and/or cooling flavors, preferably lower the levels of MSG and/or coolant such as menthol in various food products to provide advantageous properties as well as economy of use.

### Summary of the Invention

The present invention has discovered that amide compounds represented by Formula I set forth below have unexpected and advantageous flavor enhancement and modification properties: 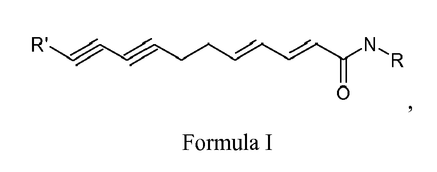 wherein R and R' is independently selected from the group consisting of H and C₁-C₁₀ linear, branched, or cyclic alkyl, alkenyl, alkynyl or aromatic groups.

One embodiment of the invention relates to a process of augmenting, enhancing or imparting a taste to a material selected from the group consisting of foodstuff, a chewing gum, a dental or oral hygiene product, and a medicinal product comprising the step of incorporating a composition comprising an olfactory effective amount of the compound of Formula I provided above; and a flavor compound selected from the group consisting of an umami compound and a cooling compound.

Another embodiment of the invention relates to a composition comprising the compound of Formula I provided above; and a flavor compound selected from the group consisting of an umami compound and a cooling compound.

Another embodiment of the invention relates to a process of augmenting, enhancing or imparting a taste to a material selected from the group consisting of foodstuff, a chewing gum, a dental or oral hygiene product, and a medicinal product comprising the step of incorporating a composition comprising an olfactory effective amount of the compound of Formula I provided above, wherein R is a C₆-C₁₀ hydrocarbon containing one phenyl group and R' is H; and a flavor compound selected from the group consisting of an umami compound and a cooling compound.

Another embodiment of the invention relates to a composition comprising the compound of Formula I provided above, wherein R is a C₆-C₁₀ hydrocarbon containing one phenyl group and R' is H; and a flavor compound selected from the group consisting of an umami compound and a cooling compound.

Another embodiment of the invention relates to a process of augmenting, enhancing or imparting a taste to a material selected from the group consisting of foodstuff, a chewing gum, a dental or oral hygiene product, and a medicinal product comprising the step of incorporating a composition comprising an olfactory effective amount of (2E,4E)-N-phenethylundeca-2,4-dien-8,10-diynamide; and an umami compound.

Another embodiment of the invention relates to a composition comprising (2E,4E)-N-phenethylundeca-2,4-dien-8,10-diynamide; and an umami compound.

Another embodiment of the invention relates to a process of augmenting, enhancing or imparting a taste to a material selected from the group consisting of foodstuff, a chewing gum, a dental or oral hygiene product, and a medicinal product comprising the step of incorporating a composition comprising an olfactory effective amount of (2E,4E)-N-phenethylundeca-2,4-dien-8,10-diynamide; and MSG.

Another embodiment of the invention relates to a composition comprising (2E,4E)-N-phenethylundeca-2,4-dien-8,10-diynamide; and MSG.

These and other embodiments of the present invention will be apparent by reading the following specification.

### Detailed Description of the Invention

It is known to those with the skill in the art that Formulas I as defined above provides the following novel compounds:

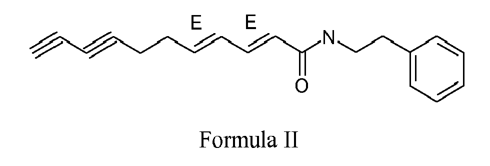

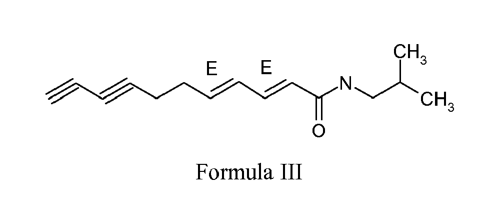

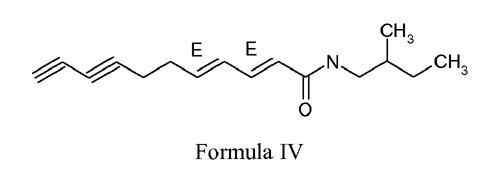

Those with the skill in the art will recognize that
Formula II represents (2E,4E)-N-phenethylundeca-2,4-dien-8,10-diynamide;
Formula III represents (2E,4E)-N-isobutylundeca-2,4-dien-8,10-diynamide; and
Formula IV represents (2E,4E)-N-(2-methylbutyl)undeca-2,4-dien-8,10-diynamide.

The compounds of the present invention can be prepared from pent-4-yn-1-ol (commercially available from Sigma-Aldrich, Inc.). The reaction steps can be depicted by general schemes shown as follows: 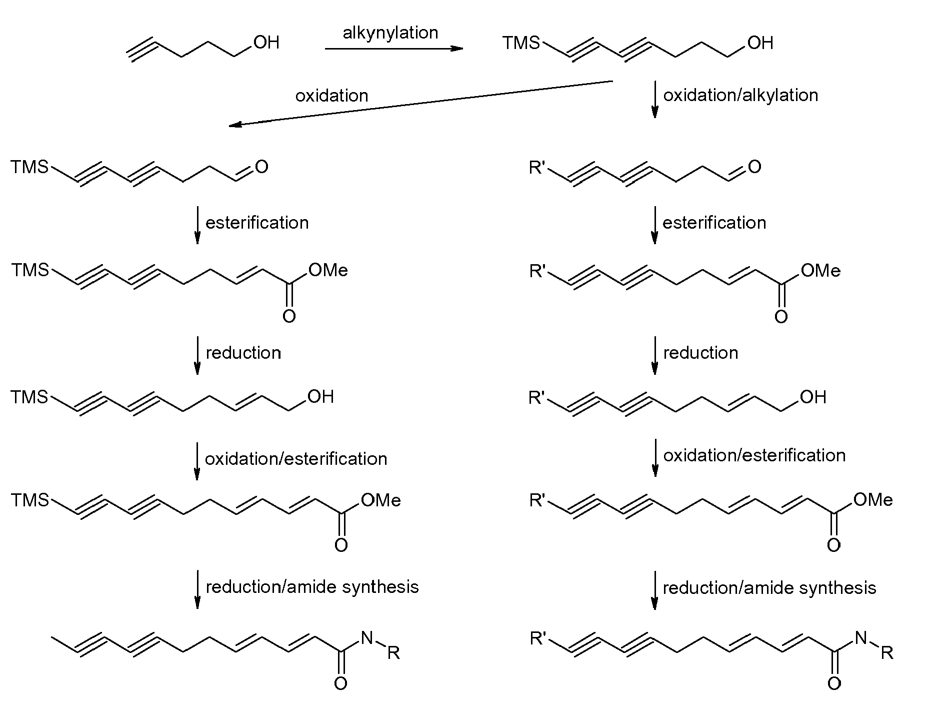 wherein R and R' are defined as above;
Me represents CH₃; and
TMS represents trimethylsilane.

Those with skill in the art will recognize that some of the intermediate compounds contain isomeric mixtures. Those with skill in the art will further recognize that those isomeric mixtures can be separated using techniques known in the art. Suitable techniques may include chromatography such as high performance liquid chromatography, referred to as HPLC, and particularly gel chromatography and solid phase microextraction, referred to as SPME. Thus, the specific isomeric compounds of the present invention will be achieved.

The compounds of the present invention are surprisingly found to have unexpected properties of enhancing umami as well as enhancing cooling in flavors, which are demonstrated to be advantageous for the use in augmenting or imparting taste enhancement or somatosensory effect to foodstuff, chewing gums, oral hygiene products, and medicinal products by providing flavor enhancement and a preferred overall flavor profile.

The compounds of the present invention can be used in combination with other flavor compounds such as umami and/or cooling compounds that are known in the art. For example, the compounds may be employed to enhance the perceived umami taste of MSG since large amount of MSG may cause adverse effect as well as allergic reactions in human. In a preferred embodiment, the compounds are used in combination with MSG in a weight ratio of at least about 1:200,000, preferably from about 1:20,000 to about 1:2, more preferably from about 1:10,000 to about 1:20, and even more preferably from about 1:2,000 to about 1:40.

As used herein, an umami compound is understood to mean a compound that exhibits umami flavor, and a cooling compound is understood to mean a compound that exhibits cooling effect.

As used herein, an olfactory effective amount is understood to mean the amount of the compound in a flavor composition that alters the characteristics of the composition, or enhances or modifies the flavor, taste, and aroma reaction contributed by another ingredient in the composition. The overall flavor, taste, and aroma effect of the composition will be the sum effect of all flavor ingredients. The olfactory effective amount will vary depending on many factors including other ingredients, their relative amounts, and the effect that is desired.

The usage level of the compounds of the present invention varies depending on the product in which the compounds are employed. Generally, the level of the compounds employed in a product is greater than about 1 part per billion by weight, preferably from about 10 parts per billion to about 100 parts per million by weight, more preferably from about 50 parts per billion to about 10 parts per million by weight.

As used herein, foodstuff includes both solid and liquid ingestible materials for man or animals, which materials usually do, but need not, have nutritional value. Thus, foodstuff includes food products, such as meats, gravies, soups, convenience foods, malt, alcoholic and other beverages, milk and dairy products, seafood, including fish, crustaceans, mollusks and the like, candies, vegetables, cereals, soft drinks, snacks, dog and cat foods, other veterinary products and the like.

When the compounds of the present invention are used in an orally consumable composition, they can be combined with conventional flavoring ingredients or adjuvants, which are well known in the art. Requirements of such flavoring ingredients and adjuvants are that: (1) they be organoleptically compatible with the compounds of the present invention whereby the flavor of the ultimate consumable composition to which the compounds are added is not detrimentally affected by the use of such flavoring ingredients and adjuvants; and (2) they be ingestible acceptable and thus nontoxic or otherwise non-deleterious. In addition, the orally consumable composition can broadly include other flavor materials, vehicles, stabilizers, thickeners, surface active agents, conditioners, and flavor intensifiers.

The following are provided as specific embodiments of the present invention. Other modifications of this invention will be readily apparent to those skilled in the art. Such modifications are understood to be within the scope of this invention. As used herein all percentages are weight percent unless otherwise noted, ppb is understood to stand for parts per billion, ppm is understood to stand for parts per million, L is understood to be liter, mL is understood to be milliliter, g is understood to be gram, mol is understood to be mole, and mmol is understood to be millimole.

### EXAMPLE I

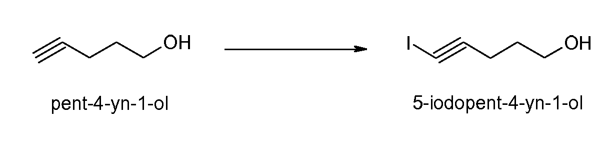

**Preparation of 5-Iodopent-4-yn-1-ol:** Potassium hydroxide (115 g, 2.051 mol, commercially available from Sigma-Aldrich, Inc.) was dissolved in water (150 ml) and cooled to 0 °C. Pent-4-yn-1-ol (69 g, 820 mmol, commercially available from Sigma-Aldrich, Inc.) was dissolved in methanol (1.125 L) and slowly added to the reaction mixture while maintaining the temperature at 0 °C. After 15-30 minutes, iodine (229 g, 902 mmol) was added in one portion and the mixture was warmed to room temperature and stirred for 3 hours. The mixture was then diluted with water (750 mL) and washed three times with diethyl ether (Et₂O, 300 mL). The organic layers were combined and concentrated *in vacuo* to provide a yellow oil. The oil was dissolved in methylene chloride (CH₂Cl₂) (300 mL), washed with brine (300 mL), dried with sodium sulfate (Na₂SO₄), and filtered. The solvent was then removed *in vacuo* to provide the crude product (170 g), which was purified by column chromatography with hexane:ethyl acetate (Hex:EtOAc) to provide 5-iodopent-4-yn-1-ol (154 g, 89% yield).
¹H NMR (500 MHz, CDCl₃) δ: 1.50 ppm (s, 1H), 1.77 ppm (pentet, 2H, J = 6.50 Hz), 2.50 ppm (t, 2H, J = 6.92 Hz), 3.74 ppm (s, 2H). EIMS *m*/*z*: M⁺ 172.

### EXAMPLE II

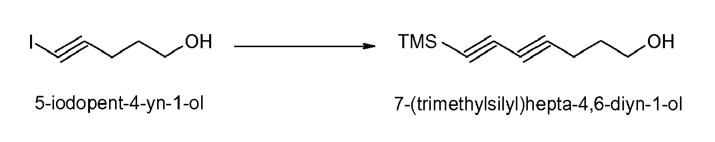

**Preparation of 7-(Trimethylsilyl)hepta-4,6-diyn-1-ol:** Ethynyltrimethylsilane (112 g, 1.143 mol, commercially available from Sigma-Aldrich, Inc.), piperidine (847 ml, 8.571 mol, commercially available from Sigma-Aldrich, Inc.), and 5-iodopent-4-yn-1-ol (120 g, 0.571 mol, prepared as above) were combined and cooled to 0 °C. Copper(I) chloride (5.66 g, 57.1 mmol, commercially available from Sigma-Aldrich, Inc.) was added in one portion. The reaction mixture was stirred with gradual warming to room temperature. After 30 minutes, the solution was quenched with saturated ammonium chloride solution (NH₄Cl) (2.5 L) and washed three times with Et₂O (300 mL). The organic layers were combined, washed twice with brine (500 mL), dried with Na₂SO₄, filtered, and concentrated *in vacuo* using a rotary evaporator. The crude product was purified by silica gel chromatography (Hex:EtOAc 6:1) to provide 7-(trimethylsilyl)hepta-4,6-diyn-1-ol (91.8 g, 86% yield).
¹H NMR (500 MHz, CDCl₃) δ: 0.18 ppm (s, 9H), 1.53 ppm (s, 1H), 1.78 ppm (pentet, 2H, J = 6.56 Hz), 2.41 ppm (t, 2H, J = 6.97 Hz), 3.74 ppm (t, 2H, J = 5.88 Hz). EIMS *m*/*z*: M⁺ 180

### EXAMPLE III

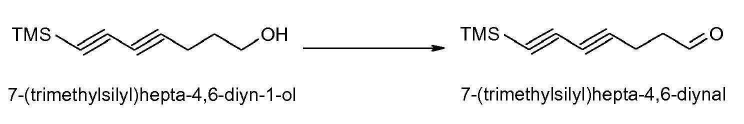

**Preparation of 7-(Trimethylsilyl)hepta-4,6-diynal:** Dimethyl sulfoxide (DMSO) (76 ml, 1.076 mol) was added dropwise at -78 °C to a solution of oxalyl chloride (47.1 ml, 538 mmol, commercially available from Sigma-Aldrich, Inc.) in CH₂Cl₂ (750 ml). The reaction mixture was stirred for 20 minutes at -78 °C. 7-(Trimethylsilyl)hepta-4,6-diyn-1-ol (50 g, 269 mmol, prepared as above) was dissolved in CH₂Cl₂ (15 mL) and slowly added. The reaction mixture was stirred for 1 hour at -78 °C. Triethylamine (225 ml, 1.614 mol, commercially available from Sigma-Aldrich, Inc.) was then added. The reaction mixture was further stirred for 80 minutes while slowly warming to room temperature. The reaction mixture was quenched with saturated NH₄Cl, separated, and the aqueous portion was back-extracted twice with CH₂Cl₂ (200 mL). The organic layers were combined, dried with Na₂SO4, filtered, and concentrated *in vacuo.* The crude was purified by column chromatography (Hex:EtOAc) to provide 7-(trimethylsilyl)hepta-4,6-diynal (22.9 g, 47.8% yield).
¹H NMR (500 MHz, CDCl₃) δ: 0.18 ppm (s, 9H), 2.60 ppm (t, 2H, J = 7.07 Hz), 2.71 ppm (t, 2H, J = 7.09 Hz), 9.78 ppm (s, 1H). EIMS *m*/*z*: M⁺ 178

### EXAMPLE IV

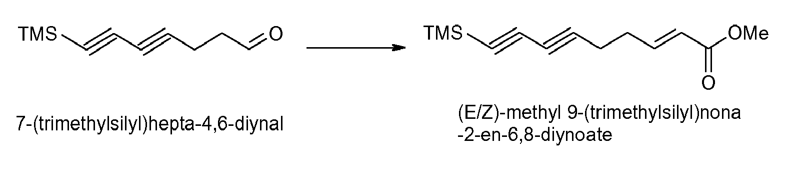

**Preparation of (E)-Methyl 9-(trimethylsilyl)nona-2-en-6,8-diynoate and (Z)-Methyl 9-(trimethylsilyl)nona-2-en-6,8-diynoate:** A solution of sodium hydride (5.89 g, 147 mmol) in tetrahydrofuran (THF) (500 ml) was cooled to 0 °C. Methyl 2-(dimethoxyphosphoryl)acetate (25.3 ml, 172 mmol, commercially available from Sigma-Aldrich, Inc.) was added dropwise and the reaction mixture was stirred for 20 minutes at 0 °C. 7-(Trimethylsilyl)hepta-4,6-diynal (17.5 g, 98 mmol, prepared as above) was dissolved in THF (50 mL), added dropwise while gradually warming to room temperature, and stirred for 1 hour. The reaction mixture was quenched with saturated NH₄Cl (500 ml). The aqueous portion was extracted three times with Et₂O (200 mL). The organic layers were combined, dried with Na₂SO₄, filtered, and concentrated *in vacuo.* The crude product had an E:Z isomeric ratio of 8:1 and was purified by column chromatography (Hex:EtOAc) to provide the products (E)-methyl 9-(trimethylsilyl)nona-2-en-6,8-diynoate (16.0 g, 69.6% yield) and (Z)-methyl 9-(trimethylsilyl)nona-2-en-6,8-diynoate (2.0 g, 8.69 % yield), respectively.

(E)-Methyl 9-(trimethylsilyl)nona-2-en-6,8-diynoate had the following NMR spectral characteristics:
¹H NMR (500 MHz, CDCl₃) δ: 0.19 ppm (s, 9H), 2.45 ppm (s, 4H), 3.74 ppm (s, 3H), 5.89 ppm (d, 1H, J = 15.57 Hz), 6.95 ppm (d, 1H, J = 15.67 Hz, of d, J = 6.18 Hz). MS *m*/*z*: M⁺ 234.

(Z)-Methyl 9-(trimethylsilyl)nona-2-en-6,8-diynoate had the following NMR spectral characteristics:
¹H NMR (500 MHz, CDCl₃) δ: 0.18 ppm (s, 9H), 2.44 ppm (t, 2H, J = 6.95 Hz), 2.88 ppm (q, 2H, J = 7.04 Hz), 3.71 ppm (s, 3H), 5.86 ppm (d, 1H, J = 11.46 Hz), 6.30 ppm (d, 1H, J = 11.41 Hz, of d, J = 7.28 Hz). EIMS *m*/*z*: M⁺ 234

### EXAMPLE V

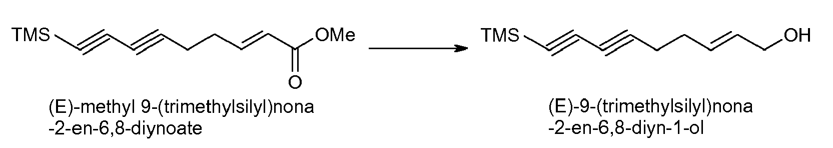

**Preparation of (E)-9-(Trimethylsilyl)nona-2-en-6,8-diyn-1-ol:** (E)-Methyl 9-(trimethylsilyl)nona-2-en-6,8-diynoate (12.7 g, 54.2 mmol, prepared as above) was dissolved in THF (400 ml) and cooled to -78 °C. Diisobutylaluminium hydride (DIBAL-H, commercially available from Sigma-Aldrich, Inc.) solution in THF (1 M, 163 ml, 163 mmol) was slowly added. The reaction mixture was stirred for 2 hours while maintaining the temperature at -78 °C. The reaction mixture was then quenched with EtOAc (600 mL) at -78 °C, warmed, and washed with HCl (1 M) and brine. The organic layers were combined, dried with Na₂SO₄, filtered, and concentrated *in vacuo.* The crude was purified by column chromatography (Hex:EtOAc) to provide (E)-9-(trimethylsilyl)nona-2-en-6,8-diyn-1-ol (8.0 g, 71.5% yield).
¹H NMR (500 MHz, CDCl₃) δ: 0.18 ppm (s, 9H), 1.34 ppm (t, 1H, J = 5.33 Hz), 2.27-2.30 ppm (m, 2H), 2.35-2.39 ppm (m, 2H), 4.12 ppm (s, 2H), 5.72 ppm (m, 2H). EIMS *m*/*z*: M⁺ 206

### EXAMPLE VI

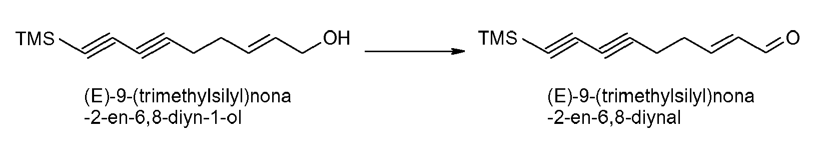

**Preparation of (E)-9-(Trimethylsilyl)nona-2-en-6,8-diynal:** DMSO (19.12 ml, 269 mmol) was added dropwise at -78 °C to a solution of oxalyl chloride (11.79 ml, 135 mmol, commercially available from Sigma-Aldrich, Inc.) in CH₂Cl₂ (250 ml). The mixture was stirred for 20 minutes. (E)-9-(Trimethylsilyl)nona-2-en-6,8-diyn-1-ol (13.9 g, 67.4 mmol, prepared as above) was dissolved in CH₂Cl₂ (15 ml) and slowly added. The reaction mixture was stirred for 1 hour. Triethylamine (56.3 ml, 404 mmol, commercially available from Sigma-Aldrich, Inc.) was added and the reaction mixture was stirred for 80 minutes while slowly warming to room temperature. The reaction mixture was quenched with saturated NH₄Cl and separated. The aqueous portion was extracted twice with CH₂Cl₂ (100 mL). The organic layers were combined, dried with Na₂SO₄, filtered, and concentrated *in vacuo.* The crude product was purified by column chromatography (Hex:EtOAc) to provide (E)-9-(trimethylsilyl)nona-2-en-6,8-diynal (10.4 g, 76% yield).
¹H NMR (500 MHz, CDCl₃) δ: 0.19 ppm (s, 9H), 2.49-2.60 ppm (m, 4H), 6.18 ppm (d, 1H, J = 15.68 Hz, of d, J = 7.76 Hz), 6.85 ppm (d, 1H, J = 15.68 Hz, of d, J = 6.42 Hz), 9.54 ppm (d, 1H,J=7.77Hz). EIMS *m*/*z*: M⁺ 204

### EXAMPLE VII

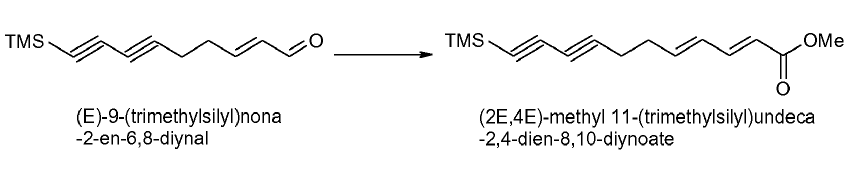

**Preparation of (2E,4E)-Methyl 11-(trimethylsilyl)undeca-2,4-dien-8,10-diynoate:** A solution of sodium hydride (3.05 g, 76 mmol) in THF (400 ml) was cooled to 0 °C. Methyl 2-(dimethoxyphosphoryl)acetate (13.10 ml, 89 mmol, commercially available from Sigma-Aldrich, Inc.) was added dropwise. The reaction mixture was stirred for 20 minutes. (E)-9-(Trimethylsilyl)nona-2-en-6,8-diynal (10.4 g, 50.9 mmol, prepared as above) was dissolved in THF (10 mL) and added dropwise while maintaining the temperature at 0 °C. The reaction mixture was then gradually warmed to room temperature and stirred for another hour. The reaction mixture was quenched with saturated NH₄Cl and separated. The aqueous portion was extracted three times with Et₂O (100 mL). The organic layers were combined, dried with Na₂SO₄, filtered, and concentrated *in vacuo.* The crude product was purified by column chromatography (Hex:EtOAc) to provide (2E,4E)-methyl 11-(trimethylsilyl)undeca-2,4-dien-8,10-diynoate (5.0 g, 38% yield).
¹H NMR (500 MHz, CDCl₃) δ: 0.19 ppm (s, 9H), 2.41 ppm (s, 4H), 3.74 ppm (s, 3H), 5.84 ppm (d, 1H, J = 15.39 Hz), 6.12 ppm (d, 1H, J = 15.23 Hz, of d, J = 5.88 Hz), 6.22 ppm (d, 1H, J = 15.07 Hz, of d, J = 10.93 Hz), 7.26 ppm (d, 1H, J = 15.27 Hz, of d, J = 10.82 Hz). EIMS *m*/*z*: M⁺ 260

### EXAMPLE VIII

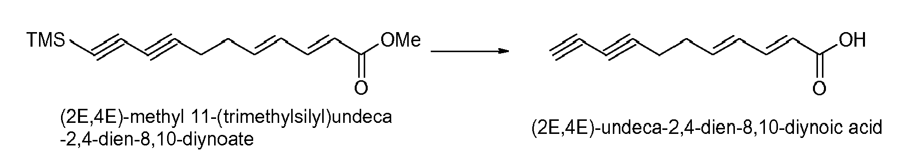

**Preparation of (2E,4E)-Undeca-2,4-dien-8,10-diynoic acid:** (2E,4E)-Methyl 11-(trimethylsilyl)undeca-2,4-dien-8,10-diynoate (3.2 g, 12.29 mmol, prepared as above) was added to sodium hydroxide solution (10%) and stirred at room temperature. The reaction was monitored by thin layer chromatography (TLC) (Hex:EtOAc). After 24 hours the reaction mixture was neutralized to pH 6.5 and extracted with EtOAc. The organic layer was washed with water and brine, dried with magnesium sulfate (MgSO₄), and concentrated *in vacuo* to provide (2E,4E)-undeca-2,4-dien-8,10-diynoic acid (2.0 g, 93% yield).
¹H NMR (500 MHz, CDCl₃) δ: 1.99 ppm (s, 1H), 2.42 ppm (s, 4H), 5.85 ppm (d, 1H, J = 15.36 Hz), 6.17 ppm (d, 1H, J = 15.33 Hz, of d, J = 5.96 Hz), 6.28 ppm (d, 1H, J = 14.99 Hz, of d, J = 10.90 Hz), 7.34 ppm (d, 1H, J = 15.89 Hz, of d, J = 10.73 Hz). EIMS *m*/*z*: M⁺ 174, APCI-MS *m*/*z*: (M⁺-H) 173

### EXAMPLE IX

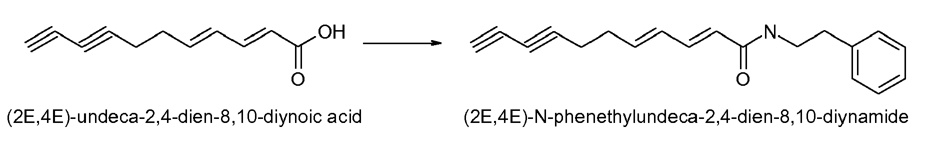

**Preparation of (2E,4E)-N-Phenethylundeca-2,4-dien-8,10-diynamide (Formula II):** (2E,4E)-Undeca-2,4-dien-8,10-diynoic acid (0.50 g, 2.87 mmol, prepared as above), triethylamine (0.639 g, 6.31 mmol, commercially available from Sigma-Aldrich, Inc.), N1-((ethylimino)methylene)-N3,N3-dimethylpropane-1,3-diamine hydrochloride (0.605 g, 3.16 mmol, commercially available from Sigma-Aldrich, Inc.), and 1H-benzo[d][1,2,3]triazol-1-ol hydrate (0.484 g, 3.16 mmol, commercially available from Sigma-Aldrich, Inc.) were dissolved in dimethylformamide (DMF) (20 ml). After 30 minutes, 2-phenylethanamine (0.417 g, 3.44 mmol, commercially available from Sigma-Aldrich, Inc.) was dissolved in DMF (1 mL) and added dropwise. The reaction was monitored by TLC. Once the reaction completed, the reaction mixture was poured over water and extracted three times with EtOAc (50mL). The organic layers were combined and washed with water (100 mL) and brine (100 ml), dried with MgSO₄, filtered, and concentrated *in vacuo.* The crude product was purified by column chromatography (Hex:EtOAc 3:1) to provide (2E,4E)-N-phenethylundeca-2,4-dien-8,10-diynamide (0.2 g, 25.1% yield).
¹H NMR (500 MHz, CDCl₃) δ: 1.98 ppm (s, 1H), 2.38 ppm (s, 2H), 2.39 ppm (s, 2H), 2.85 ppm (t, 2H, J = 6.89 Hz), 3.61 ppm (q, 2H, J = 6.56 Hz), 5.47 ppm (br. s, 1H), 5.73 ppm (d, 1H, J = 15.00 Hz), 6.01-6.08 ppm (m, 1H), 6.17 ppm (d, 1H, J = 15.11 Hz, of d, J= 10.80 Hz), 7.14-7.33 ppm (m, 6H). APCI-MS *m*/*z*: (M⁺+ H) 278.

### EXAMPLE X

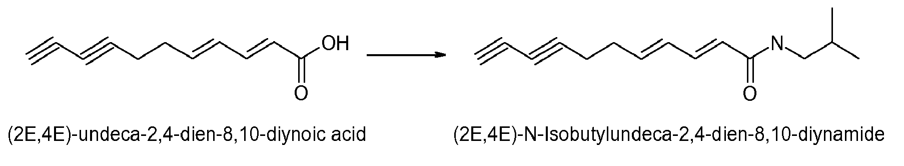

**Preparation of (2E,4E)-N-Isobutylundeca-2,4-dien-8,10-diynamide (Formula III):** (2E,4E)-Undeca-2,4-dien-8,10-diynoic acid (0.50 g, 2.87 mmol, prepared as above), triethylamine (0.639 g, 6.31 mmol), N1-((ethylimino)methylene)-N3,N3-dimethylpropane-1,3-diamine hydrochloride (0.605 g, 3.16 mmol), and 1H-benzo[d][1,2,3]triazol-1-0l hydrate (0.484 g, 3.16 mmol) were dissolved in DMF (20 ml). After 30 minutes, 2-methylpropan-1-amine (0.252 g, 3.44 mmol, commercially available from Sigma-Aldrich, Inc.) was dissolved in DMF (1 mL) and added dropwise. The reaction was monitored by TLC. Once the reaction completed, the reaction mixture was poured over water and extracted three times with EtOAc (50 mL). The organic layers were combined and washed with water (100 mL) and brine (100 ml), dried with MgSO₄, filtered, and concentrated *in vacuo.* The crude product was purified by column chromatography (Hex:EtOAc 3:1) to provide (2E,4E)-N-isobutylundeca-2,4-dien-8,10-diynamide (0.20 g, 30.4% yield).
¹H NMR (500 MHz, CDCl₃) δ: 0.93 ppm (d, 6H, J = 6.70 Hz), 1.80 ppm (m, 1H, J = 6.71 Hz), 1.98 ppm (s, 1H), 2.39 ppm (s, 4H), 3.17 ppm (t, 2H, J = 6.46 Hz), 5.45 ppm (m, 1H), 5.80 ppm (d, 1H, J = 14.65 Hz), 6.07 ppm (m, 1H), 6.20 ppm (d, 1H, J = 15.08 Hz, of d, J= 10.76 Hz), 7.18 ppm (d, 1H, J = 15.07 Hz, of d, J = 11.08 Hz). APCI-MS *m*/*z*: (M⁺+H) 230.

### EXAMPLE XI

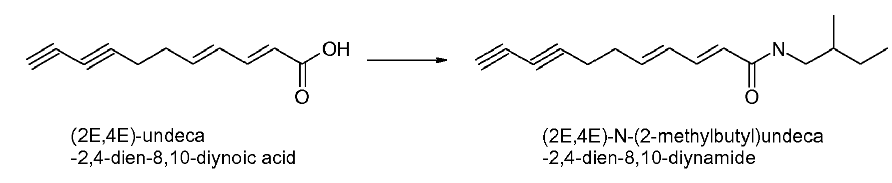

**Preparation of (2E,4E)-N-(2-Methylbutyl)undeca-2,4-dien-8,10-diynamide (Formula IV):** (2E,4E)-Undeca-2,4-dien-8,10-diynoic acid (0.50 g, 2.87 mmol, prepared as above), triethylamine (0.639 g, 6.31 mmol), N1-((ethylimino)methylene)-N3,N3-dimethylpropane-1,3-diamine hydrochloride (0.605 g, 3.16 mmol), and 1H-benzo[d][1,2,3]triazol-1-ol hydrate (0.484 g, 3.16 mmol) were dissolved in DMF (20 ml). After 30 minutes, 2-methylbutan-1-amine (0.300 g, 3.44 mmol, commercially available from Sigma-Aldrich, Inc.) was dissolved in DMF (1 mL) and added dropwise. The reaction was monitored by TLC. Once the reaction completed, the reaction mixture was poured over water and extracted three times with EtOAc (50 mL). The organic layers were combined and washed with water (100 mL) and brine (100 ml), dried with MgSO₄, filtered, and concentrated *in vacuo.* The crude product was purified by column chromatography (Hex:EtOAc 3:1) to provide (2E,4E)-N-(2-methylbutyl)undeca-2,4-dien-8,10-diynamide (0.20 g, 28.6% yield).
¹H NMR (500 MHz, CDCl₃) δ: 0.91 ppm (d, 3H, J = 6.66 Hz), 0.91 ppm (t, 3H, J = 7.36 Hz), 1.14-1.20 ppm (m, 1H), 1.36-1.45 ppm (m, 1H), 1.52-1.61 ppm (m, 1H), 1.98 ppm (s, 1H), 2.39 ppm (s, 4H), 3.13-3.19 ppm (m, 1H), 3.26-3.32 ppm (m, 1H), 5.44 ppm (br. s, 1H), 5.80 ppm (d, 1H, J = 14.99 Hz), 6.06 ppm (m, 1H), 6.20 ppm (d, 1H, J = 15.09 Hz, of d, J= 10.88 Hz), 7.18 ppm (d, 1H, J = 14.90 Hz, of d, J = 10.86 Hz). APCI-MS *m*/*z*: (M⁺+H) 244.

### EXAMPLE XII

The flavor compositions of MSG (0.02%) combined with (2E,4E)-N-phenethylundeca-2,4-dien-8,10-diynamide (Formula II) at different concentrations were evaluated by a trained sensory panel using an intensity scale of 0 to 5, where 0 = none, 1 = minimal, 3 = moderate, and 5 = intense. Averaged sensory scores were reported in the following:

| **Composition** | **Concentration (Formula II)** | **Flavor Profile** | **Flavor Intensity** |
|---|---|---|---|
| MSG | 0 | umami | 1.0 |
| MSG/Formula II | 100 ppb | Slight off-note, delocalized, fuller umami, lingered umami, slightly bitter in the back of throat, faster onset of umami sensation. | 2.0 |
| MSG/Formula II | 250 ppb | Stronger umami, enhanced mouthcoating, extended duration of mouthcoating, enhanced umami in the back of mouth/throat. | 3.0 |
| MSG/Formula II | 500 ppb | Very strong umami, heavy umami, full profile, slight sweetness of MSG perceived, enhanced umami in the back of mouth/throat. | 3.5 |
| MSG/Formula II | 1 ppm | Heavy umami, full profile, slight sweetness of MSG perceived, enhanced mouthcoating, increased salivation, enhanced umami in the back of mouth/throat. | 3.7 |
| MSG/Formula II | 5 ppm | Heavy umami, full profile, slight sweetness of MSG perceived, boosted umami intensity, slight tingle, delocalized umami, increased salivation, enhanced umami in the back of mouth/throat (when comparable to the 250 ppb group), slightly soapy, some potentiation of umami on sides of tongue. | 4.3 |

Formula II enhanced the umami flavor of MSG. The MSG/Formula II composition (500 ppb) provided the strongest umami taste in the back of mouth/throat when compared with other groups.

### EXAMPLE XIII

The MSG-inosine monophosphate (IMP) mixture (0.01% MSG and 0.005% IMP), an umami composition, was combined with Formulas II-IV at different concentrations and evaluated by a trained sensory panel. Sensory evaluation was reported in the following:

| **Composition** | **Flavor Profile** |
|---|---|
| MSG-IMP | Heavy umami, full profile, slight sweetness of MSG perceived, enhanced umami in the back of mouth/throat. |
| MSG-IMP/Formula II | Formula II (500 ppb, 1 ppm, and 5 ppm) enhanced the umami flavor of the MSG-IMP mixture. |
| MSG-IMP/Formula III | Formula III (500 ppb) enhanced the umami flavor of the MSG-IMP mixture. |
| MSG-IMP/Formula IV | Formula III (500 ppb and 1 ppm) enhanced the umami flavor of the MSG-IMP mixture. |

Formulas II, III, and IV all enhanced the umami flavor of the MSG-IMP mixture. Formula II exhibited the strongest enhancement at 500 ppb.

### EXAMPLE XIV

The cooling compound menthol (5 ppm) was combined with Formulas II-IV at different concentrations and evaluated by a trained sensory panel. Sensory evaluation was reported in the following:

| **Composition** | **Flavor Profile** |
|---|---|
| Menthol/Formula II | Formula II (500 ppb, 1 ppm, and 5 ppm) enhanced the cooling effect of menthol, off-notes perceived at 5 ppm. |
| Menthol/Formula III | Formula III (500 ppb) enhanced the cooling effect of menthol when compared with Formula III at 1 ppm and 5 ppm. |
| Menthol/Formula IV | Formula III (500 ppb) slightly increased the cooling effect of menthol. |

### EXAMPLE XV

The cooling compound WS3 (5 ppm) was combined with Formulas II-IV at different concentrations and evaluated by a trained sensory panel. Sensory evaluation was reported in the following:

| **Composition** | **Flavor Profile** |
|---|---|
| WS3/Formula II | Formula II (5 ppm) enhanced the cooling effect of WS3. |
| WS3/Formula III | Formula III (500 ppb and 1 ppm) enhanced the cooling effect and longevity of WS3 when compared with Formula III at 5 ppm. |
| WS3/Formula IV | Formula III (500 ppb and 1 ppm) increased the cooling effect of WS3 and provided faster onset of cooling when compared with Formula IV at 5 ppm. |

## Claims

1. A composition comprising a compound of Formula I: 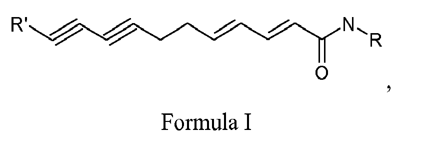 wherein R and R' is independently selected from the group consisting of H and C₁-C₁₀ linear, branched, or cyclic alkyl, alkenyl, alkynyl or aromactic groups; and
a flavor compound selected from the group consisting of an umami compound and a cooling compound.

2. The composition of claim 1, wherein R is a C₆-C₁₀ hydrocarbon containing one phenyl group and R' is H.

3. The composition of claim 1 or of claim 2 further comprising a material selected from the group consisting of foodstuff, a chewing gum, a dental or oral hygiene product, and a medicinal product.

4. The composition of claim 3, wherein the compound is provided at a level of greater than about 1 part per billion by weight.

5. The composition of claim 3, wherein the compound is provided at a level of from about 10 parts per billion to about 100 parts per million by weight.

6. The composition of claim 3, wherein the compound is provided at a level of from about 50 parts per billion to about 10 parts per million by weight.

7. A composition comprising (2E,4E)-N-phenethylundeca-2,4-dien-8,10-diynamide and an umami compound.

8. A process of augmenting, enhancing or imparting a taste to a material selected from the group consisting of foodstuff, a chewing gum, a dental or oral hygiene product, and a medicinal product comprising the step of incorporating a composition comprising an olfactory effective amount of a compound of Formula I: 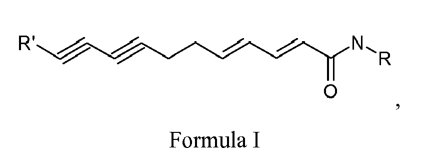 wherein R and R' is independently selected from the group consisting of H and C₁-C₁₀ linear, branched, or cyclic alkyl, alkenyl, alkynyl or aromactic groups; and
a flavor compound selected from the group consisting of an umami compound and a cooling compound.

9. The process of claim 8, wherein the compound is (2E,4E)-N-phenethylundeca-2,4-dien-8,10-diynamide and the flavor compound is an umami compound.

10. The composition of claim 7 further comprising a material selected from the group consisting of foodstuff, a chewing gum, a dental or oral hygiene product, and a medicinal product.

11. The composition of claim 7, wherein the umami compound is MSG, or the process of claim 9 wherein the umami compound is MSG.

12. The composition or the process of claim 11, wherein (2E,4E)-N-phenethylundeca-2,4-dien-8,10-diynamide and MSG have a weight ratio of at least about 1:200,000.

13. The composition or the process of claim 11, wherein (2E,4E)-N-phenethylundeca-2,4-dien-8,10-diynamide and MSG have a weight ratio of from about 1:20,000 to about 1:2.

14. The composition or the process of claim 11, wherein (2E,4E)-N-phenethylundeca-2,4-dien-8,10-diynamide and MSG have a weight ratio of from about 1:10,000 to about 1:20.

15. The composition or the process of claim 11, wherein (2E,4E)-N-phenethylundeca-2,4-dien-8,10-diynamide and MSG have a weight ratio of from about 1:2,000 to about 1:40.
